# EUROPEAN PATENT APPLICATION

(11) **EP 3 412 656 A1**
(43) Date of publication of application: **12.12.2018**
(21) Application number: 17175063.1
(22) Date of filing: 08.06.2017
(51) Int. Cl.: C07D 213/73, A61P 21/04

(54) **CRYSTALLINE FORMS OF AMIFAMPRIDINE DIHYDROCHLORIDE**

(71) Applicant: Alfred E. Tiefenbacher (GmbH & Co. KG), 22767 Hamburg (DE)
(72) Inventor: Staver, Ruslan, 22607 Hamburg (DE); Kupka, Anna, 44803 Bochum (DE)
(74) Representative: Hamm&Wittkopp Patentanwälte PartmbB

(57) **Abstract**

The present invention relates to crystalline forms of amifampridine dihydrochloride having an irregular, not needle-like crystal habit and their use for the treatment of disorders like the Lambert-Eaton myasthenic syndrome (LEMS).

## Description

The present invention relates to crystalline forms of amifampridine dihydrochloride and their use in therapy.

Amifampridine (3,4-diaminopyridine, 3,4-DAP) has been widely used as an *ad-hoc* hospital preparation in the treatment of Lambert-Eaton myasthenic syndrome (LEMS). Since the on-demand preparations of amifampridine exhibited variability and a lack of reliability in the quality of the drug product, a tablet was developed containing 10 mg of amifampridine as a phosphate salt. This tablet is commercially available under the tradename Firdapse^{®} for the symptomatic treatment of LEMS. Firdapse^{®} is an immediate-release tablet containing microcrystalline cellulose anhydrous colloidal silica and calcium stearate as pharmaceutical excipients, whereby the tablet is prepared by direct compression.

The preparation of amifampridine and its monohydrochloride salt is described in EP 0 159 112. US 2004/0106651 relates to the manufacture of the phosphate and tartrate salt of amifampridine and their use for the treatment of botulism, myasthenia, myasthenic syndromes and fatigue related to a neurological pathology, e.g. multiple sclerosis or amyotrophic lateral sclerosis (ALS). The crystal structure and solid state properties of amifampridine phosphate is described in Cryst, Growth Des. 2013, 13, 708-715. In this article, it is also mentioned that six amifampridine salts had been prepared (hydrogen chloride, hydrogen bromide, sulfate, dihydrogen phosphate, tartrate and benzoate), which are more stable than the free base, since the involvement of the lone electron pair on the pyridine nitrogen in the charge-assisted hydrogen bond reduces the possibility of oxidation for amifampridine. Among these salts, only two, namely the dihydrogen phosphate and hydrogen tartrate salt, have been selected for medical use because they appear physically and chemically stable in time, possess little hygroscopicity, have excellent solubility in water and have an acceptable pH in saturated solution.

The preparation and the crystalline structure of the dihydrochloride salt of amifampridine is disclosed in Acta Cryst. 2009, E65, o131, and in the supporting information. The salt was prepared by preparing a solution of amifampridine and HCl in ethanol and allowing the solution to slowly evaporate; the crystalline form of amifampridine dihydrochloride obtained by crystallization from ethanol is hereinafter designated as form 1. The prior art crystals of form 1 are needle-like crystals, and it is commonly known that such crystals show poor flowability properties; the needles have a length up to 1800 µm, which requires micronization for the drug to be processed into solid dosage forms.

It was an object of the present invention to improve the processability of amifampridine dihydrochloride. This object is solved by the subject matter as defined in the claims.

The amifampridine dihydrochloride salt of the present invention is a crystalline material, whereby the crystals have an irregular shape. These crystals do not show a needle-like habit, and they may form aggregates. Typically, the crystals and aggregates of the present invention have a length-wise diameter in the range of 3-300 µm, preferably 10-250 µm. The salt of the present invention is a free-flowing material that can be easily filtered and processed into a solid unit dosage form without the need of micronization. Thus, the salt of the present invention is in particular suitable for preparing a solid unit dosage form, e.g. a tablet, through dry-granulation or direct compression.

The dihydrochloride salt of amifampridine of the present invention is obtainable by precipitation from an aqueous solution containing the salt, in which the aqueous solution and an anti-solvent are combined. Slow crystallization of amifampridine dihydrochloride from an ethanolic solution provides the prior art form 1, while the relatively fast crystallization (precipitation) by using an anti-solvent gives the irregular crystals of the present invention. In the course of the inventor's investigation of the solid-state properties of amifampridine dihydrochloride, four additional crystalline forms have been detected, which are hereinafter designated as forms 2-5.

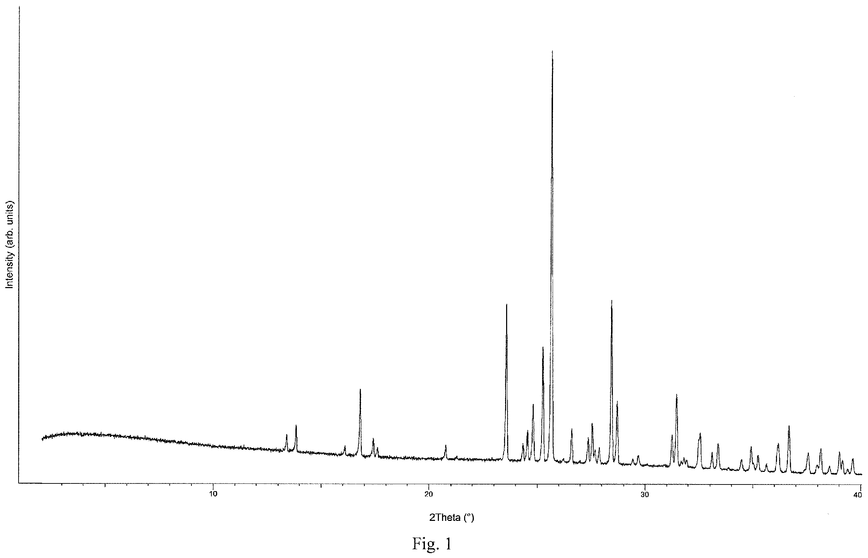

- Figure 1:: XRPD of form 1 (transmission mode; Cu-Kα1 = 1.54059 Å).

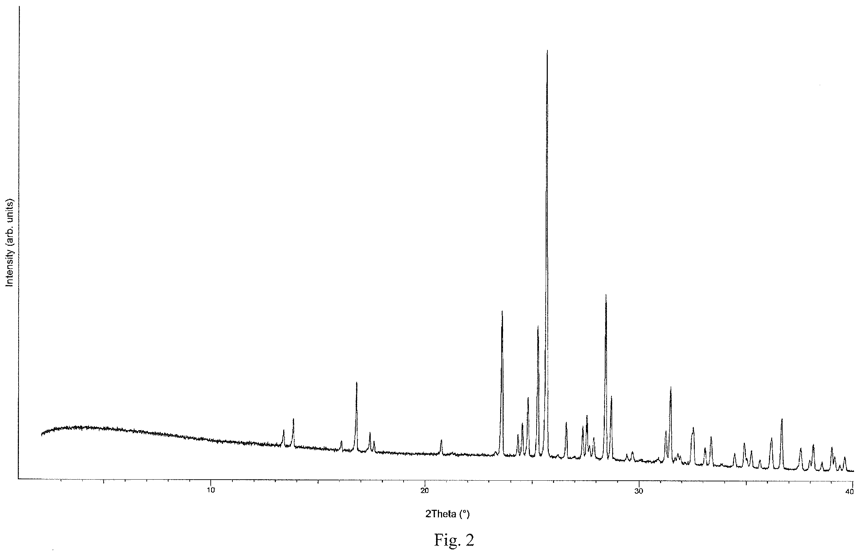

- Figure 2:: XRPD of form 1 with traces of form 3 (transmission mode; Cu-Kα1=1.54059 Å)

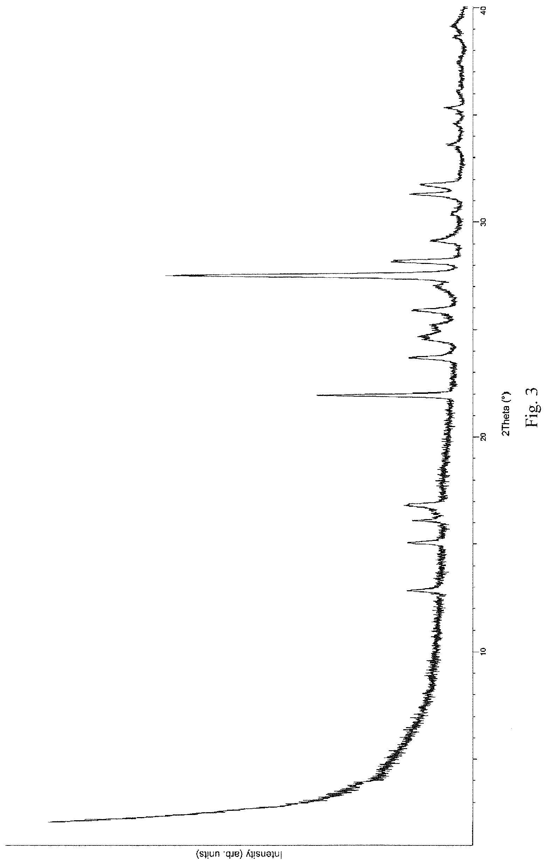

- Figure 3:: XRPD of form 2 (reflection mode; Cu-Kα1 = 1.54059 Å)

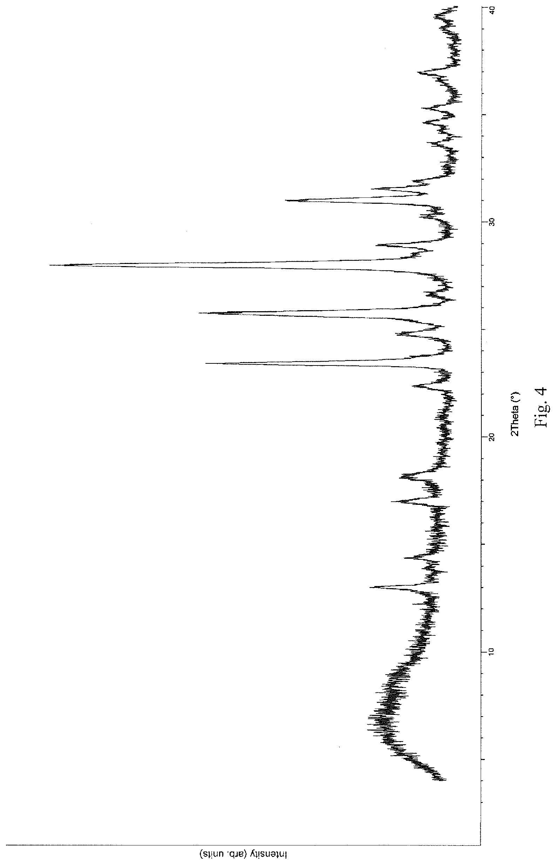

- Figure 4:: XRPD of form 3 (reflection mode; Cu-Kα1/2 = 1.5418 Å)

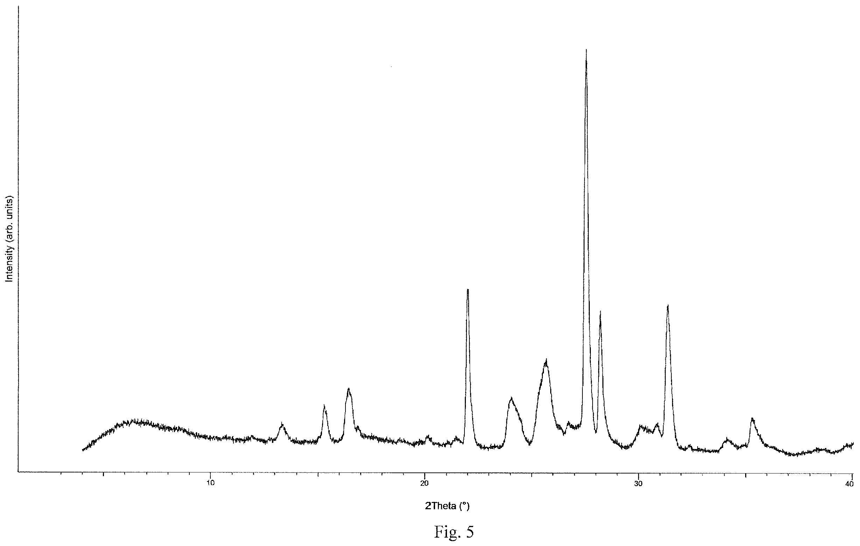

- Figure 5:: XRPD of form 4 (reflection mode; Cu-Kα1/2 = 1.5418 Å)

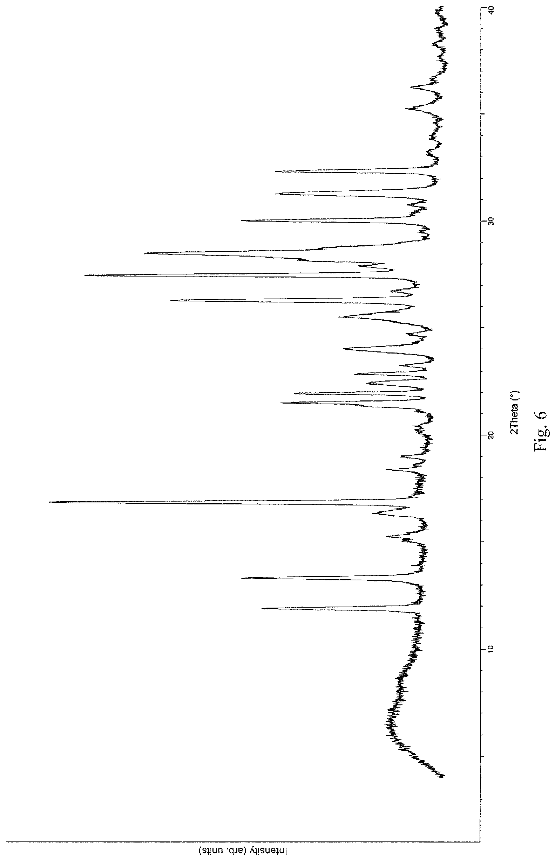

- Figure 6:: XRPD of a mixture of forms 4 and 5 (reflection mode; Cu-Kα1/2 = 1.5418 Å)
- Figure 7:: Microscopic image of form 1 with traces of form 3 in silicone oil
- Figure 8:: Microscopic image of the prior art form 1 in silicone oil
- Figure 9:: Microscopic image of form 2 in silicone oil
- Figure 10:: Microscopic image of form 3 in silicone oil
- Figure 11:: Microscopic image of form 4 in silicone oil
- Figure 12:: Microscopic image of a mixture of forms 4 and 5 in silicone oil

The crystalline form 1 with traces of form 3 is obtained by preparing a solution of amifampridine dihydrochloride in water and adding an antisolvent like acetone, ethylacetate or n-hexane, preferably acetone. The obtained crystalline material is non-hygroscopic with a maximum mass increase of about 0.28 % as determined by volumetric dynamic vapor sorption (volumetric DVS); the moisture uptake is partly reversible. The differential scanning calorimetry (DSC) thermogram of the form 1 with traces of form 3 revealed a melting point at 233.1°C and a small endothermal peak (about 15 J/g) at 160°C.

The form 1 of amifampridine dihydrochloride is characterized by the following peaks of the powder X-ray diffractogram (Cu-Kα1 = 1.54059 Å): 16.80, 23.57, 24.80, 25.26, 25.67, 27.56, 28.44, 28.70, 31.46, 36.66 ± 0.20° 20. The powder X-ray diffractogram of form 1 contains additional peaks at (Cu-Kα1 = 1.54059 Å): 13.39, 13.83, 16.10, 17.40, 17.60, 20.75, 24.33, 24.54, 26.60, 27.37, 27.68, 27.88, 29.43, 29.68, 31.24, 31.68, 31.80, 31.92, 32.54, 33.09, 33.37, 34.47, 34.91, 35.25, 35.64, 36.18, 37.56, 37.98, 38.14, 38.56, 39.02, 39.16, 39.40, 39.63 ± 0.20° 2θ. The trace amounts of the form 3 are indicated by the following peaks in the powder X-ray diffractogram (Cu-Kα1 = 1.54059 Å): 23.29, 30.88 ± 0.20° 20. The complete powder XRD-pattern of form 1 is shown in table 1 below.

**Table 1. Form 1**

| **Peak** | **2Theta (°)** | **2Theta (°)** | **rel. Intensity (%)** |
|---|---|---|---|
| | | *Strongest peaks* | |
| 1 | 13.39 | | 4.1 |
| 2 | 13.83 | | 6.7 |
| 3 | 16.10 | | 2.4 |
| 4 | 16.80 | 16.80 | 16.3 |
| 5 | 17.40 | | 4.6 |
| 6 | 17.60 | | 2.3 |
| 7 | 20.75 | | 3.4 |
| 8 | 23.57 | 23.57 | 38.0 |
| 9 | 24.33 | | 4.2 |
| 10 | 24.54 | | 7.4 |
| 11 | 24.80 | 24.80 | 13.8 |
| 12 | 25.26 | 25.26 | 27.9 |
| 13 | 25.67 | 25.67 | 100.0 |
| 14 | 26.60 | | 8.3 |
| 15 | 27.37 | | 6.2 |
| 16 | 27.56 | 27.56 | 9.9 |
| 17 | 27.68 | | 3.2 |
| 18 | 27.88 | | 3.8 |
| 19 | 28.44 | 28.44 | 40.0 |
| 20 | 28.70 | 28.70 | 15.5 |
| 21 | 29.43 | | 1.5 |
| 22 | 29.68 | | 2.3 |
| 23 | 31.24 | | 7.6 |
| 24 | 31.46 | 31.46 | 17.8 |
| 25 | 31.68 | | 1.5 |
| 26 | 31.80 | | 2.4 |
| 27 | 31.92 | | 1.6 |
| 28 | 32.54 | | 8.7 |
| 29 | 33.09 | | 3.9 |
| 30 | 33.37 | | 5.9 |
| 31 | 34.47 | | 2.7 |
| 32 | 34.91 | | 5.9 |
| 33 | 35.25 | | 3.8 |
| 34 | 35.64 | | 1.9 |
| 35 | 36.18 | | 7.0 |
| 36 | 36.66 | 36.66 | 11.5 |
| 37 | 37.56 | | 4.9 |
| 38 | 37.98 | | 2.1 |
| 39 | 38.14 | | 6.0 |
| 40 | 38.56 | | 1.9 |
| 41 | 39.02 | | 5.1 |
| 42 | 39.16 | | 3.4 |
| 43 | 39.40 | | 1.2 |
| 44 | 39.63 | | 3.6 |

The complete powder XRD-pattern of form 1 with traces of form 3 is shown in table 2 below.

**Table 2. Form 1 with traces of form 3**

| **Peak** | **2Theta (°)** | **2Theta (°)** | **rel. Intensity** |
|---|---|---|---|
| | | *Strongest peaks* | **(%)** |
| 1 | 13.40 | | 3.9 |
| 2 | 13.84 | | 7.0 |
| 3 | 16.10 | | 2.3 |
| 4 | 16.80 | 16.80 | 16.9 |
| 5 | 17.41 | | 4.5 |
| 6 | 17.60 | | 2.6 |
| 7 | 20.76 | | 3.6 |
| 8 | 23.29 | | 0.9 (Form 3) |
| 9 | 23.58 | 23.58 | 35.6 |
| 10 | 24.34 | | 5.2 |
| 11 | 24.54 | | 8.2 |
| 12 | 24.80 | 24.80 | 14.3 |
| 13 | 25.26 | 25.26 | 32.0 |
| 14 | 25.67 | 25.67 | 100.0 |
| 15 | 26.60 | | 8.8 |
| 16 | 27.37 | | 8.0 |
| 17 | 27.56 | 27.56 | 10.6 |
| 18 | 27.68 | | 3.4 |
| 19 | 27.88 | | 5.1 |
| 20 | 28.44 | 28.44 | 40.6 |
| 21 | 28.70 | 28.70 | 15.6 |
| 22 | 29.43 | | 1.4 |
| 23 | 29.69 | | 2.4 |
| 24 | 30.88 | | 0.8 (Form 3) |
| 25 | 31.25 | | 7.7 |
| 26 | 31.46 | 31.46 | 18.6 |
| 27 | 31.68 | | 1.4 |
| 28 | 31.81 | | 2.4 |
| 29 | 31.93 | | 1.8 |
| 30 | 32.54 | | 8.8 |
| 31 | 33.10 | | 4.2 |
| 32 | 33.37 | | 6.8 |
| 33 | 34.48 | | 3.3 |
| 34 | 34.91 | | 5.7 |
| 35 | 34.96 | | 2.9 |
| 36 | 35.24 | | 4.1 |
| 37 | 35.64 | | 1.9 |
| 38 | 36.18 | | 6.9 |
| 39 | 36.67 | 36.67 | 12.2 |
| 40 | 37.56 | | 5.2 |
| 41 | 37.98 | | 2.4 |
| 42 | 38.15 | | 6.2 |
| 43 | 38.56 | | 2.1 |
| 44 | 39.02 | | 5.7 |
| 45 | 39.14 | | 3.1 |
| 46 | 39.39 | | 1.3 |

The form 2 of amifampridine dihydrochloride is obtained by dissolving the salt in water and adding the aqueous solution to an anti-solvent, e.g. acetone, ethylacetate, n-hexane, preferably acetone. In slurry experiments, the form 2 did not convert into form 1. However, in solid state, form 2 slowly converted into form 1 during storage at room temperature for at least two months. The DSC thermogram of form 2 revealed a melting point at 231.9°C and a very small endothermal peak (about 3 J/g) at 137.3°C.

The form 2 of amifampridine dihydrochloride is characterized by the following peaks of the powder X-ray diffractogram (Cu-Kα1 = 1.54059 Å): 12.82, 15.05, 16.81, 21.94, 23.68, 25.89, 27.52, 28.18, 31.31, 31.75 ± 0.20° 20. The powder X-ray diffractogram of form 2 contains additional peaks at (Cu-Kα1 = 1.54059 Å): 16.10, 24.58, 24.71, 25.20, 26.96, 29.18, 30.39, 33.58, 35.33 ± 0.20° 2θ. The complete powder XRD-pattern of form 2 is shown in table 3 below.

**Table 3. Form 2**

| **Peak** | **2Theta (°)** | **2Theta (°)** | **rel. Intensity** |
|---|---|---|---|
| | | *Strongest peaks* | **(%)** |
| 1 | 12.82 | 12.82 | 12.7 |
| 2 | 15.05 | 15.05 | 12.5 |
| 3 | 16.10 | | 9.3 |
| 4 | 16.81 | 16.81 | 14.2 |
| 5 | 21.94 | 21.94 | 47.7 |
| 6 | 23.68 | 23.68 | 15.7 |
| 7 | 24.58 | | 8.7 |
| 8 | 24.71 | | 11.2 |
| 9 | 25.20 | | 6.2 |
| 10 | 25.89 | 25.89 | 13.7 |
| 11 | 26.96 | | 6.0 |
| 12 | 27.52 | 27.52 | 100.0 |
| 13 | 28.18 | 28.18 | 21.1 |
| 14 | 29.18 | | 9.9 |
| 15 | 30.39 | | 3.1 |
| 16 | 31.31 | 31.31 | 17.9 |
| 17 | 31.75 | 31.75 | 14.0 |
| 18 | 33.58 | | 4.2 |
| 19 | 35.33 | | 6.5 |

Stirring form 1, optionally with traces of form 3, in acetone for several days at room temperature or at higher temperatures, e.g. at 50°C, preferably for at least seven days, yielded form 3.

The form 3 of amifampridine dihydrochloride is characterized by the following peaks of the powder X-ray diffractogram (Cu-Kα1/2 = 1.5418 Å): 12.98, 16.98, 18.15, 23.40, 24.80, 25.73, 27.97, 28.92, 30.99, 31.53 ± 0.20° 2θ. The powder X-ray diffractogram of form 3 contains additional peaks at (Cu-Kα1/2 = 1.5418 Å): 14.36, 22.35, 26.65, 30.22, 31.88, 33.66, 34.24, 34.62, 35.28, 36.97, 39.58 ± 0.20° 2θ. The complete powder XRD-pattern of form 3 is shown in table 4 below.

**Table 4. Form 3**

| **Peak** | **2Theta (°)** | **2Theta (°)** | **rel. Intensity** |
|---|---|---|---|
| | | *Strongest peaks* | **(%)** |
| 1 | 12.98 | 12.98 | 16.6 |
| 2 | 14.36 | | 8.5 |
| 3 | 16.98 | 16.98 | 11.6 |
| 4 | 18.15 | 18.15 | 10.3 |
| 5 | 22.35 | | 9.0 |
| 6 | 23.40 | 23.40 | 59.9 |
| 7 | 24.80 | 24.80 | 13.3 |
| 8 | 25.73 | 25.73 | 61.4 |
| 9 | 26.65 | | 4.9 |
| 10 | 27.97 | 27.97 | 100.0 |
| 11 | 28.92 | 28.92 | 16.4 |
| 12 | 30.22 | | 7.4 |
| 13 | 30.99 | 30.99 | 38.0 |
| 14 | 31.53 | 31.53 | 19.1 |
| 15 | 31.88 | | 8.7 |
| 16 | 33.66 | | 5.1 |
| 17 | 34.24 | | 2.7 |
| 18 | 34.62 | | 6.2 |
| 19 | 35.28 | | 6.4 |
| 20 | 36.97 | | 9.6 |
| 21 | 39.58 | | 5.1 |

By using hot-stage microscopy and DSC, it could be revealed that the endothermal peak that appeared at 160°C in the DSC of form 1 (with traces of form 3) indicates a phase transition. Heating form 1, optionally with traces of form 3, to 170°C (DSC) with a heating rate of 10 K/min gives the form 4 of amifampridine dihydrochloride.

The form 4 of amifampridine dihydrochloride is characterized by the following peaks of the powder X-ray diffractogram (Cu-Kα1/2 = 1.5418 Å): 15.31, 16.45, 22.00, 24.03, 25.65, 27.52, 28.19, 30.84, 31.36, 35.33 ± 0.20° 20. The powder X-ray diffractogram of form 4 contains additional peaks at (Cu-Kα1/2 = 1.5418 Å): 13.36, 16.83, 20.15, 21.48, 26.75, 30.15, 32.38, 34.15 ± 0.20° 20. The complete powder XRD-pattern of form 4 is reported in table 5 below.

**Table 5. Form 4**

| **Peaks** | **2Theta (°)** | **2Theta (°)** | **rel. Intensity** |
|---|---|---|---|
| | | *Strongest peaks* | **(%)** |
| 1 | 13.36 | | 4.4 |
| 2 | 15.31 | 15.31 | 9.0 |
| 3 | 16.45 | 16.45 | 13.7 |
| 4 | 16.83 | | 2.8 |
| 5 | 20.15 | | 1.8 |
| 6 | 21.48 | | 2.2 |
| 7 | 22.00 | 22.00 | 39.9 |
| 8 | 24.03 | 24.03 | 12.5 |
| 9 | 25.65 | 25.65 | 21.1 |
| 10 | 26.75 | | 5.3 |
| 11 | 27.52 | 27.52 | 100.0 |
| 12 | 28.19 | 28.19 | 33.8 |
| 13 | 30.15 | | 5.2 |
| 14 | 30.84 | 30.84 | 5.8 |
| 15 | 31.36 | 31.36 | 36.3 |
| 16 | 32.38 | | 1.0 |
| 17 | 34.15 | | 2.7 |
| 18 | 35.33 | 35.33 | 8.4 |

If form 1, optionally with traces of form 3, is heated to 190°C (DSC) with a heating rate of 10 K/min, a mixture of the form 4 with another form, designated as form 5, is obtained.

The mixture of forms 4 and 5 of amifampridine dihydrochloride is characterized by the following peaks of the powder X-ray diffractogram (Cu-Kα1/2 = 1.5418 Å): 11.88, 13.31, 16.84, 21.49, 26.27, 27.44, 28.47, 30.01, 31.27, 32.31 ± 0.20° 2θ. The powder X-ray diffractogram of the mixture contains additional peaks at (Cu-Kα1/2 = 1.5418 Å): 15.26, 16.34, 18.37, 18.97, 21.92, 22.41, 22.86, 23.24, 23.97, 24.69, 25.53, 26.73, 27.88, 30.71, 35.23, 36.23 ± 0.20° 2θ. The complete XRD-pattern of this mixture is shown in table 6 below.

**Table 6. Mixture of form 5 and form 4**

| **Peak** | **2Theta (°)** | **2Theta (°)** | **rel. Intensity** |
|---|---|---|---|
| | | *Strongest peaks* | **(%)** |
| 1 | 11.88 | 11.88 | 42.3 |
| 2 | 13.31 | 13.31 | 47.5 |
| 3 | 15.26 | | 9.8 |
| 4 | 16.34 | | 13.0 |
| 5 | 16.84 | 16.84 | 100.0 |
| 6 | 18.37 | | 10.3 |
| 7 | 18.97 | | 6.6 |
| 8 | 21.49 | 21.49 | 39.6 |
| 9 | 21.92 | | 36.2 |
| 10 | 22.41 | | 16.9 |
| 11 | 22.86 | | 19.5 |
| 12 | 23.24 | | 8.2 |
| 13 | 23.97 | | 22.7 |
| 14 | 24.69 | | 5.8 |
| 15 | 25.53 | | 22.2 |
| 16 | 26.27 | 26.27 | 68.0 |
| 17 | 26.73 | | 9.3 |
| 18 | 27.44 | 27.44 | 90.8 |
| 19 | 27.88 | | 16.5 |
| 20 | 28.47 | 28.47 | 74.9 |
| 21 | 30.01 | 30.01 | 48.4 |
| 22 | 30.71 | | 5.7 |
| 23 | 31.27 | 31.27 | 40.6 |
| 24 | 32.31 | 32.31 | 42.1 |
| 25 | 35.23 | | 8.7 |
| 26 | 36.23 | | 8.3 |

The amifampridine dihydrochloride salt of the present invention is suitable for the preparation of solid and liquid pharmaceutical compositions, e.g. a tablet. In particular, the salts can be used in the preparation of a tablet through direct compression. The dihydrochloride salt of amifampridine of the present invention can be used for the treatment of botulism, myasthenia gravis, congenital myasthenic syndromes (CMS), Lambert-Eaton myasthenic syndrome (LEMS) or fatigue related to a neurological pathology, such as multiple sclerosis and amyotrophic lateral sclerosis (ALS). The invention is further illustrated by reference to the following examples.

### Examples

### Analytical methods:

**XRPD** (transmission mode): For Powder X-Ray Diffraction (PXRD) the sample was placed into a standard glass capillary (Ø = 0.7 mm), after careful grinding. The measurement was performed at room temperature with a Bruker D8 Advance Diffractometer (Cu-Kα1 = 1.54059 Å, Johansson primary beam monochromator, position sensitive detector) in transmission mode with rotation of the sample. Data were collected in a two-theta range of 3-50°. The tube voltage and current were set to 40 kV and 40 mA, respectively.

**XRPD** (reflection mode): For Powder X-Ray Diffraction (PXRD) the sample was placed onto a special zero-background silicon wafer, after careful grinding.
1. The measurement was performed at room temperature with a Bruker D2 Phaser Diffractometer (Cu-Kα1/2 = 1.5418 Å, position sensitive detector) in reflection (Bragg-Brentano) mode with axial rotation of the sample. Data were collected in a two-theta range of 5-50°. The tube voltage and current were set to 30 kV and 10 mA, respectively.
2. The measurement was performed at room temperature with a Bruker D8 Advance Diffractometer (Cu-Kα1 = 1.54059 Å, Johansson primary beam monochromator, position sensitive detector) in reflection (Bragg-Brentano) mode with axial rotation of the sample. Data were collected in a two-theta range of 3-50°. The tube voltage and current were set to 40 kV and 40 mA, respectively.

**DSC:** DSC data were obtained on Netzsch Phoenix DSC 204 F1. Approximately 5-15 mg of each sample was placed into a DSC pan and weight accurately recorded. Sealed aluminum pans with one pinhole were used for analysis. The samples were heated under nitrogen atmosphere at a rate of 10°C/min.

**Microscopy:** Microscopic images were taken with the help of an Leica DMRB microscope. The samples were placed, in pure silicone oil, on a microscope slide and examined, under polarizable light, regarding the crystallite size and habit.

**DVS** (volumetric): Volumetric DVS measurements were carried out with a Belsorp Aqua instrument. The sample was placed in a conditioned atmosphere at 25°C. Approximately 100 mg sample was used. The humidity was set between 5 and 90 % relative humidity (RH) in approximately 10% RH steps.

### Comparative example 1. Preparation of amifampridine dihydrochloride by crystallization from an aqueous ethanolic solution

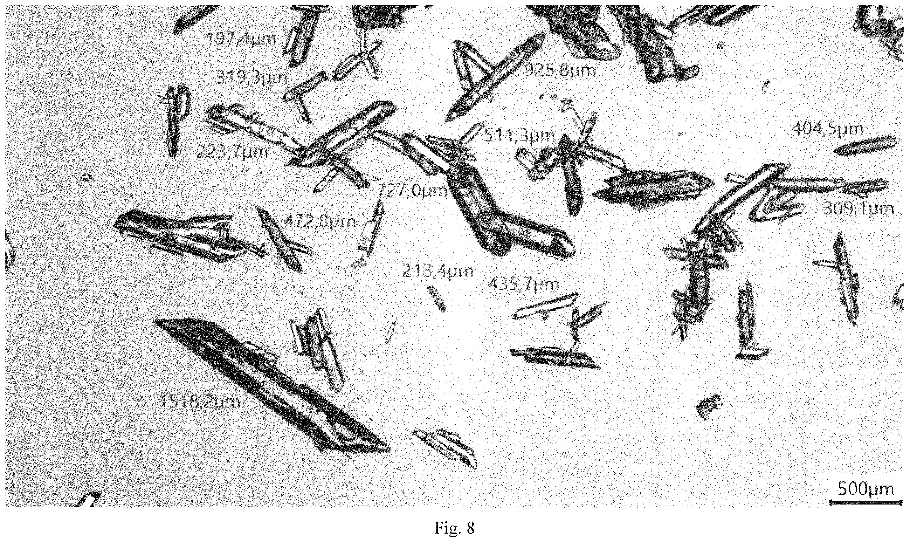

10 g amifampridine were dissolved in 68 ml hydrochloric acid (10 %). The solution was stirred at room temperature for 30 min. 250 ml ethanol were added, and the obtained solution was concentrated by applying vacuum (95 mbar) until the solvent ceased to be carried over. The obtained suspension was diluted with 100 ml ethanol, and the solvent was again removed by applying vacuum (95 mbar) until the solvent ceased to be carried over. The resulting suspension was diluted with 40 ml ethanol, and the obtained needle-like crystals (see Fig. 8) were filtered off and dried; yield: 12.02 g (72%).

¹H-NMR (D₂O, 300 MHz): δ = 6.95 (dd, 1H, H-5, ³*J*₁ = 3.3 Hz, ³*J*₂ = 0.9 Hz), 7.91 (dd, 1H, H-6, ³*J*₁ = 3.3 Hz, ³*J*₂ = 0.6 Hz), 8.05 (d, 1H, H-2, ³*J* = 0.9 Hz).

### Example 1. Preparation of amifampridine dihydrochloride form 1 with traces of form 3

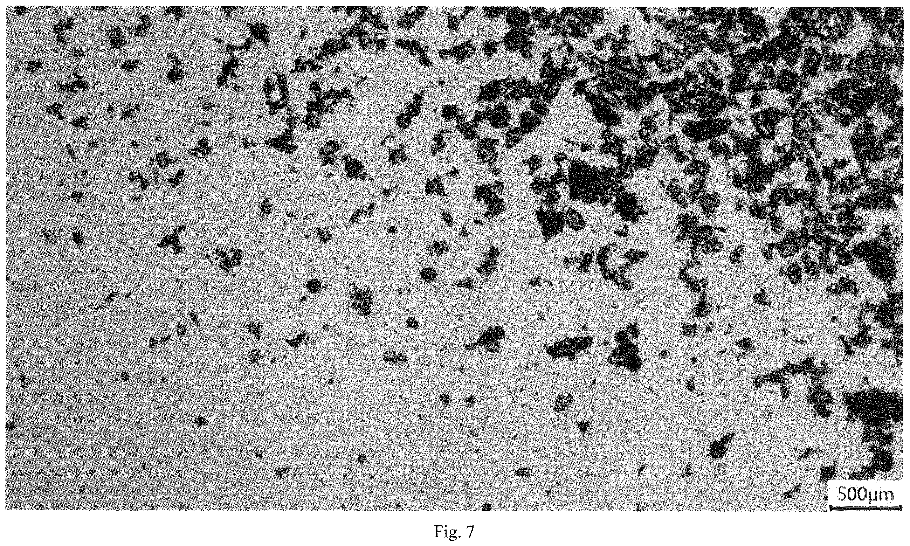

In 500 ml round-bottom flask 41 ml of deionized water are mixed with 28 ml of 25 % hydrochloric acid. The 10 grams of amifampridine are added portionwise to the mixture. After complete addition the pale yellow solution is stirred intensively at room temperature for 1 hour. 350 ml of acetone is then added to the solution under stirring. A white precipitate crystallizes out of cloudy suspension. The obtained solid is filtered off, washed twice with 50 ml of acetone and dried under vacuum at 50°C to yield 13.1 g (79%) of amifampridine dihydrochloride form 1 with traces of form 3 as irregular crystals (see Fig. 7).

### Example 2. Preparation of amifampridine dihydrochloride form 2

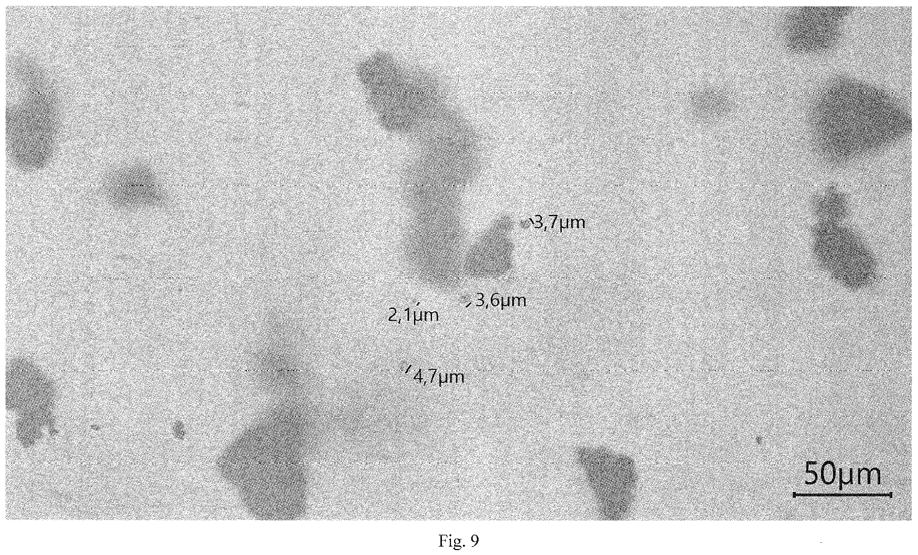

In 2 liter two-neck round-bottom flask 590 ml of deionized water are mixed with 590 ml of 25% hydrochloric acid. The 210 grams of amifampridine are added portionwise to the mixture. A mild exothermic reaction is observed (temperature raises up to 35°C). After complete addition the pale yellow solution is stirred intensively (210 rpm) at room temperature for 1 hour. The solution is then slowly added to 10.5 L of acetone under stirring (130 rpm). Total addition time is 30 minutes. A white precipitate crystallizes out of cloudy suspension. The obtained solid is filtered off, washed twice with 1 L of acetone and dried under vacuum at 50°C to yield 324 g (92%) of amifampridine dihydrochloride form 2 as irregular crystals (see Fig. 9).

### Example 3. Preparation of amifampridine dihydrochloride form 3

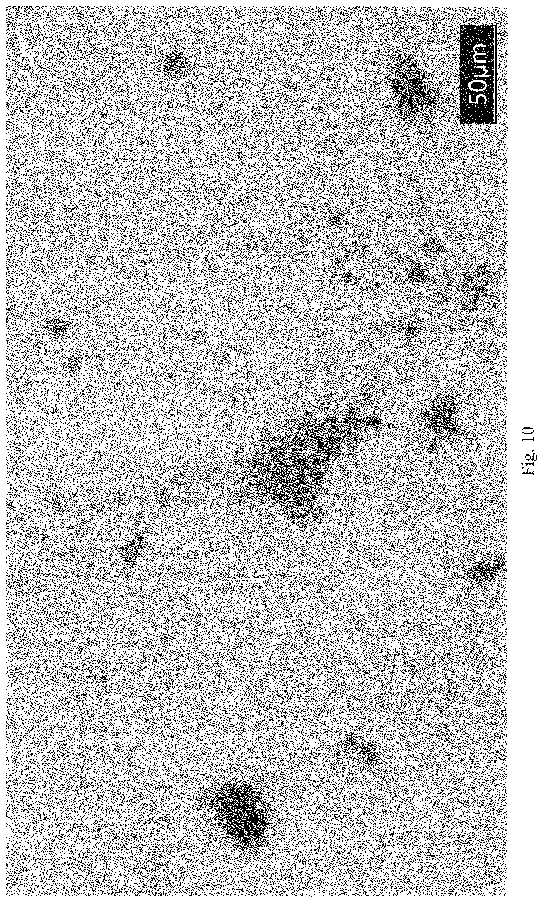

100 mg of the amifampridine dihydrochloride prepared in example 1 (form 1 with traces of form 3) were suspended in 500 µl acetone. The suspension was stirred at 50°C for 7 days. The solid was filtered off and dried to yield amifampridine dihydrochloride form 3 as irregular crystals (see Fig. 10).

### Example 4. Preparation of amifampridine dihydrochloride form 4

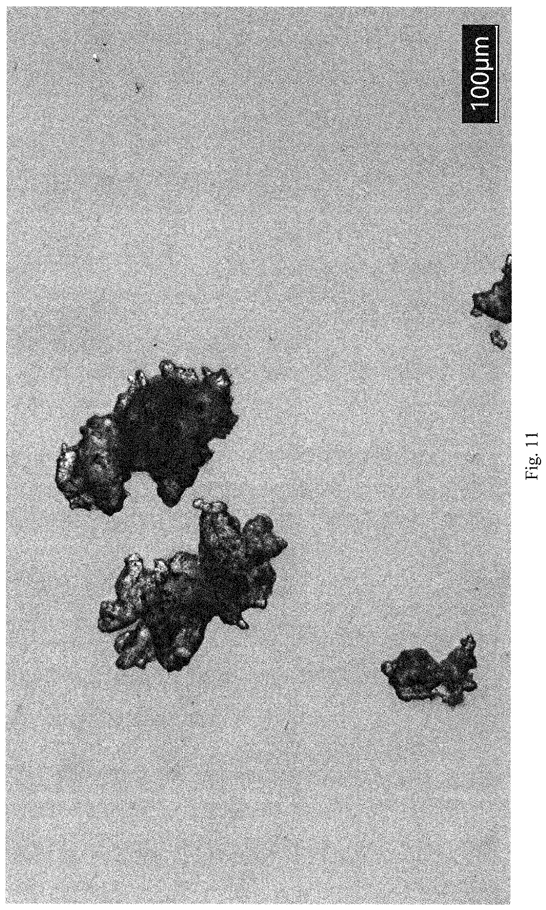

5-15 mg of amifampridine dihydrochloride form 1 (with traces of form 3) were heated from room temperature to 170°C (DSC) with a heating rate of 10 K/min, which gave form 4 as irregular crystals (see Fig. 11).

### Example 5. Preparation of amifampridine dihydrochloride form 5 in admixture with form 4

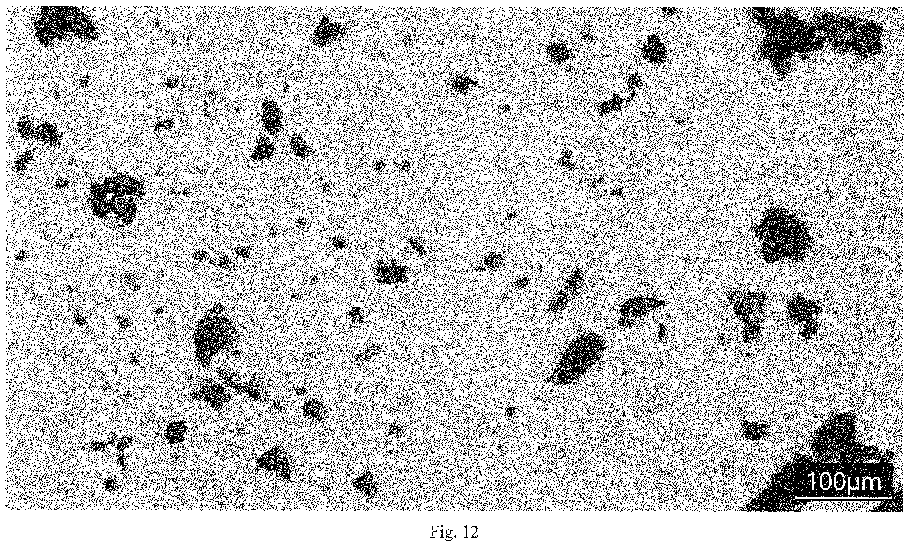

5-15 mg of amifampridine dihydrochloride form 1 (with traces of form 3) were heated from room temperature to 190°C (DSC) with a heating rate of 10 K/min, which gave the form 5 in admixture with form 4 as irregular crystals (see Fig. 12).

## Claims

1. Dihydrochloride salt of amifampridine, wherein the salt is obtainable by precipitation from an aqueous solution containing the dihydrochloride salt of amifampridine by combining the aqueous solution and an anti-solvent.

2. The salt according to claim 1, **characterized by** the following peaks of the powder X-ray diffractogram (Cu-Kα1 = 1.54059 Å): 16.80, 23.57, 24.80, 25.26, 25.67, 27.56, 28.44, 28.70, 31.46, 36.66 ± 0.20° 20.

3. The salt according to claim 1, **characterized by** the following peaks of the powder X-ray diffractogram (Cu-Kα1 = 1.54059 Å): 23.29, 30.88 ± 0.20° 20.

4. The salt according to claim 2 or 3, **characterized by** the following peaks of the powder X-ray diffractogram (Cu-Kα1 = 1.54059 Å): 13.39, 13.83, 16.10, 17.40, 17.60, 20.75, 24.33, 24.54, 26.60, 27.37, 27.68, 27.88, 29.43, 29.68, 31.24, 31.68, 31.80, 31.92, 32.54, 33.09, 33.37, 34.47, 34.91, 35.25, 35.64, 36.18, 37.56, 37.98, 38.14, 38.56, 39.02, 39.16, 39.40, 39.63 ± 0.20° 20.

5. The salt according to claim 1, **characterized by** the following peaks of the powder X-ray diffractogram (Cu-Kα1 = 1.54059 Å): 12.82, 15.05, 16.81, 21.94, 23.68, 25.89, 27.52, 28.18, 31.31, 31.75 ± 0.20° 20.

6. The salt according to claim 5, **characterized by** the following peaks of the powder X-ray diffractogram (Cu-Kα1 = 1.54059 Å): 16.10, 24.58, 24.71, 25.20, 26.96, 29.18, 30.39, 33.58, 35.33 ± 0.20° 2θ.

7. The salt according to claim 1, **characterized by** the following peaks of the powder X-ray diffractogram (Cu-Kα1/2 = 1.5418 Å): 12.98, 16.98, 18.15, 23.40, 24.80, 25.73, 27.97, 28.92, 30.99, 31.53 ± 0.20° 2θ.

8. The salt according to claim 7, **characterized by** the following peaks of the powder X-ray diffractogram (Cu-Kα1/2 = 1.5418 Å): 14.36, 22.35, 26.65, 30.22, 31.88, 33.66, 34.24, 34.62, 35.28, 36.97, 39.58 ± 0.20° 2θ.

9. The salt according to claim 1, **characterized by** the following peaks of the powder X-ray diffractogram (Cu-Kα1/2 = 1.5418 Å): 15.31, 16.45, 22.00, 24.03, 25.65, 27.52, 28.19, 30.84, 31.36, 35.33 ± 0.20° 2θ.

10. The salt according to claim 9, **characterized by** the following peaks of the powder X-ray diffractogram (Cu-Kα1/2 = 1.5418 Å): 13.36, 16.83, 20.15, 21.48, 26.75, 30.15, 32.38, 34.15 ± 0.20° 2θ.

11. The salt according to claim 1, **characterized by** the following peaks of the powder X-ray diffractogram (Cu-Kα1/2 = 1.5418 Å): 11.88, 13.31, 16.84, 21.49, 26.27, 27.44, 28.47, 30.01, 31.27, 32.31 ± 0.20° 2θ.

12. The salt according to claim 11, **characterized by** the following peaks of the powder X-ray diffractogram (Cu-Kα1/2 = 1.5418 Å): 15.26, 16.34, 18.37, 18.97, 21.92, 22.41, 22.86, 23.24, 23.97, 24.69, 25.53, 26.73, 27.88, 30.71, 35.23, 36.23 ± 0.20° 20.

13. A solid pharmaceutical composition containing a dihydrochloride salt of amifampridine according to any one of claims 1-12.

14. Use of a dihydrochloride salt of amifampridine according to any one of claims 1-12 for the preparation of a liquid or solid pharmaceutical composition.

15. A dihydrochloride salt of amifampridine according to any one of claims 1-12 for use in the treatment of botulism, myasthenia gravis, congenital myasthenic syndromes (CMS), Lambert-Eaton myasthenic syndrome (LEMS) or fatigue related to a neurological pathology, such as multiple sclerosis and amyotrophic lateral sclerosis (ALS).
